# EUROPEAN PATENT SPECIFICATION

(11) **EP 1 605 958 B1**
(45) Date of publication and mention of the grant of the patent: **12.07.2006**
(21) Application number: 04713858.1
(22) Date of filing: 24.02.2004
(51) Int. Cl.: A61K 36/185

(54) **COMPOSITIONS OF NATURAL PRODUCTS AND USE THEREOF**
ZUSAMMENSETZUNGEN AUS NATURPRODUKTEN UND VERWENDUNG DAFÜR
COMPOSITIONS A BASE DE PRODUITS NATURELS ET UTILISATION CORRESPONDANTE

(30) Priority: 21.03.2003 GB 0306568
(43) Date of publication of application: 21.12.2005
(73) Proprietor: UNILEVER PLC, London, Greater London EC4P 4BQ (GB); UNILEVER N.V., 3013 AL Rotterdam (NL)
(72) Inventor: Yates, Paula Rachel, Bedfordshire MK44 1LQ (GB)
(74) Representative: Hugot, Alain
(86) International application number: PCT/EP2004/001785
(87) International publication number: WO 2004/082697

(56) References cited:
- WO-A-02/087517
- KEIL D ET AL: "Phytoestrogenes as anti-aging agents" AKTUELLE DERMATOLOGIE 2002 GERMANY, vol. 28, no. 3, 2002, pages 69-73, XP009031640 ISSN: 0340-2541
- DATABASE WPI Section Ch, Week 199504 Derwent Publications Ltd., London, GB; Class B02, AN 1995-027623 XP002283452 & JP 06 312924 A (ASAHI BREWERIES LTD) 8 November 1994 (1994-11-08)
- HESSE R ET AL: "[Demonstration of phytoestrogens in feed plants and hops by means of receptor test]" ZENTRALBLATT FUR VETERINARMEDIZIN. REIHE A. 1981, vol. 28, no. 6, 1981, pages 422-454, XP009031839 ISSN: 0514-7158

## Description

### Field of the Invention

The present invention relates to compositions comprising a combination of hops isoalpha acids and particular isoflavones.

### Background to the Invention

Isoflavones are known as compounds that can be applied to prevent or treat many health deficiencies or to achieve certain health effects not directly related with a health deficiency. For example, they are known to achieve benefits in the women's health area, in particular for postmenopausal women. Health effects that are also attributed to isoflavones include skin anti-ageing effects and anti-inflammatory effects.

Hop materials have been used for many years to impart a distinctive, bitter flavour to brewed beverages. Hop plants are in the Cannabinaceae family. Hop materials used in the production of beer and other brewed beverages are primarily derived from a plant in the genus *Humulus* known as *Humulus lupulus L*. The key flavouring ingredients obtained from hop plants reside within cone-like structures which are harvested and used in manufacturing brewed beverages. The primary flavouring ingredients in hop cones are compounds known as alpha-acids, which are converted to isoalpha acids during the boiling process.

### Summary of the Invention

We have now found that the isoalpha acids found in hops and hop extracts can act synergistically in combination with certain isoflavones, in particular in down-regulating the production of prostaglandin E₂, part of the inflammatory response, following oxidative stress. Accordingly, a combination of hops isoalpha acids and isoflavones can be used to achieve anti-ageing and anti-inflammatory effects.

Accordingly, in a first aspect, the present invention provides a composition comprising hops isoalpha acids and one or more isoflavones selected from genistein, genistin, daidzein, daidzin, glycitein and glycitin, wherein the weight ratio of hops isoalpha acid extract to isoflavones is from 1:50 to 50:1, calculated as aglucon.

Preferably, the composition comprises genistein/genistin and daidzein/daidzin, preferably present in a weight ratio of genistein/genistin to daidzein/daidzin of from 2:1 to 1:2, calculated as aglucon, more preferably from 2:1 to 1:1 It is also preferred that the composition comprises glycitein and/or glycitin.

Preferably the isoflavones are derived from leguminous plants, more preferably soy.

In a preferred embodiment, the hops isoalpha acids are selected from unreduced isoalpha acids, dihydro-isoalpha acids and tetrahydro-isoalpha acids, and mixtures thereof.

In a second aspect, the present invention provides a dosage form comprising a composition of the invention, wherein the dosage form comprises from 10 mg to 200 mg of said hops isoalpha acids and from 10 mg to 200 mg of said isoflavones. Preferably the dosage form is an oral dosage form such as a tablet.

In a third aspect, the present invention provides a food product which comprises a composition of the invention. Preferably the amount of said hops isoalpha acids and said isoflavones present in the food product is from 20% to 100% of the total recommended daily intake (= RDI amount) of the hops isoalpha acids and isoflavones per serving.

In a preferred embodiment, the food product contains 20 mg to 400 mg of a combination of said hops isoalpha acids and said isoflavones, per recommended serving.

The present invention also provides a composition comprising a combination of hops isoalpha acids and one or more isoflavones selected from genistein, genistin, daidzein, daidzin, glycitein and glycitin for use in promoting the formation of collagen and/or decorin in the skin of an animal or human; for use in reducing the effects of ageing on the skin of an animal or human; and/or for use in treating or preventing the effects of inflammation in an animal or human.

In a related aspect, the present invention provides the use of a health composition comprising a composition or food product of the invention wherein the composition or food product is applied to achieve skin benefits and skin related effects such as anti-ageing effects or for promoting the formation of collagen or for promoting the decorin formation in the skin. Also provided is the use of said health composition wherein the composition is applied for the production of a functional food with anti-inflammatory properties.

### Detailed Description of the Invention

Unless defined otherwise, all technical and scientific terms used herein have the same meaning as commonly understood by one of ordinary skill in the art.

### Isoalpha acids

Hop materials have been used for many years to impart a distinctive, bitter flavour to brewed beverages. Hop plants are in the Cannabinaceae family. Hop materials used in the production of beer and other brewed beverages are primarily derived from a plant in the genus *Humulus* known as *Humulus lupulus L*. The key flavouring ingredients obtained from hop plants reside within cone-like structures which are harvested and used in manufacturing brewed beverages. The primary flavouring ingredients in hop cones involve materials known as "alpha acids".

Hops contain alpha acids, beta acids and gamma acids. The most important are the alpha acids, or humulones, which are characterised as humulone, cohumulone, ad humulone, pre humulone and post humulone. The relative amount of ad humulone is fairly constant between varieties whilst the relative amounts of humulone and cohumulone are variety dependent.

The majority of alpha acids present in hops, with their approximate proportions (% by weight) in parentheses, are Humulone (35-70%), Cohumulone (20-65%), Adhumulone (10-15%), Prehumulone (1-10%), and Posthumulone (1-3%) - see US Patent No. 5,478,580. These analogues differ from one another in the nature of an acyl side chain.

Alpha acids can be isomerised to form isoalpha acids, which are the form used as flavouring agents in the production of brewed beverages. The term "isoalpha acids" encompasses both the cis and trans isomerised forms of alpha acids. In addition to the unreduced forms of isoalpha acids, there are three types of reduced isoalpha acids, namely dihydro-isoalpha acids (DHIA), also known as "rho acids", tetrahydro-isoalpha acids (THIA), and hexahydro-isoalpha acids (HHIA). Accordingly, the term "isoalpha acids" encompasses both unreduced and reduced forms. Isoalpha acid preparations in the context of the present invention comprise one or more of the isomeric forms of the five major alpha acids found in hops described above, i.e. humulone, cohumulone, ad humulone, pre humulone and post humulone, preferably isomeric forms of all five.

Hops alpha acids are typically obtained in the form of a liquid hop extract. Liquid hop extracts are commercial products which are well known in the art, and are produced by chemical extraction of hop cones with selected solvent materials to remove alpha acids therefrom. In the past, extraction was undertaken using organic solvents including but not limited to methylene chloride, hexane, and methanol. However, recent technical advances have resulted in the development of extraction procedures which involve the use of liquid carbon dioxide or supercritical carbon dioxide, Thus, it is preferred that isoalpha acids according to the present invention are derived from a CO₂ hop extract, such as a liquid CO₂ or supercritical CO₂ hop extract, since the extraction method does not involve the use of any organic solvents, which is preferred when the extracts are to be used subsequently for cosmetic or pharmaceutical applications. Liquid CO₂ hop extract is commercially available from a variety of sources, including but not limited to Pfizer, Inc. and John I. Haas Co., US. Information regarding the production of hop extracts is provided in Hough, J. S., et al., Malting and Brewing Science, Vol. 2, Chapman and Hall, Ltd., Cambridge (1982), pp. 403-405.

Alpha acids can, if desired, be separated from other components such as beta acids and/or the non-acidic fraction, for example by the process described in US Patent No. 4,666,731.

There are numerous methods in which the isomerisation of alpha acids in hop materials (e.g. cones and extracts) can be achieved. For example, hops materials (e.g. hops or hop extracts) can be boiled in highly alkaline solutions (e.g. NaOH). When this process is used, care must be taken to avoid pH levels above about 9.0 since degradation of the resulting isoalpha acids may take place at such levels.

Another method for the production of isoalpha acids involves the use of metallic isomerisation materials - see for example US Patent No. 5,015,491 which describes a process wherein a hop extract is combined with a solid alkaline or alkaline earth metal salt (e.g. MgO) at a temperature of at least about 80°C. Another method is described in US Patent No. 5,478,580. Hops extracts containing isoalpha acids can be obtained commercially from Botanix Ltd., UK ('Isohops').

The reduced forms of isoalpha acids, DHIA and HHIA, can be obtained by treatment of isoalpha acids with borohydrides such as sodium borohydride and/or potassium borohydride. US 3,965,188 describes the production of DHIA from alpha acids by a simultaneous isomerisation/reduction process. This process results in the cis form whereas US 6,583,322 describes a process for producing a high ratio of trans to cis stereoisomers by separate isomerisation and reduction steps. HHIAs can also be obtained by reduction of THIAs.

Another reduced form of isoalpha acids, THIAs, can be produced from hops alpha acids or beta acids by hydrogenation in the presence of a noble metal catalyst, such as palladium - see for example US Patent No. 5,013,571 and US Patent No. 5,600,012 as well as Hay and Homiski, 1991, "Efficient One-Step Preparation of the Beer Additive Tetrahydroisoalpha-Acids," J. Agric. Food Chem. 39: 1732-1734.

Reduced forms of isoalpha acids are also available from commercial sources such as Botanix Ltd., UK and Pfizer, Inc., US.

The hops isoalpha acids that can be used according to the invention are suitably derived from natural sources as described above, for example as hops extracts. Hops contain, *inter alia,* alpha, beta and gamma acids. Isoalpha acids are a particular isomeric form of the alpha acids. The hops isoalpha acids are commercially available as purified isohop extract from Botanix Ltd. The hop acid part of the soft resin fraction contains the related alpha acids and beta acids. The gamma resins are hard, and contribute nothing to the brew. The most important are the alpha acids, or humulones, which are characterised as humulone, cohumulone, ad humulone, pre humulone and post humulone. The relative amount of ad humulone is fairly constant between varieties whilst the relative amounts of humulone and cohumulone are variety dependent.

The isoalpha acid hop extracts are commercially available in forms which may actually contain a mixture of molecular species. Typically, the isoalpha acid hop extracts comprise at least 10% w/w isoalpha acids in the reduced and/or unreduced forms described above.

The use of isolated, i.e. purified, isoalpha acid hop extracts avoids the inclusion of the potentially oestrogenic compounds found in hops. Consequently, it is preferred that the isoalpha acids, and hops extracts comprising isoalpha acids, are substantially free of oestrogenic compounds, such as 8-prenylnaringenin, i.e. contain less than 0.1 wt% oestrogenic compounds, more preferably less than 0.01 wt% oestrogenic compounds.

It is also preferred that hops isoalpha acids preparations are substantially free of hops beta acids and/or non-isomerised hops alpha acids, i.e. they contain less than 1% beta acids and/or less than 1% alpha acids, more preferably less than 0.1% beta acids and/or less than 0.1% alpha acids.

### Isoflavones

Isoflavones are polyphenolic compounds. Isoflavones are found in nature in both unglycosylated form (aglucons) and in glycosylated forms (glucons), especially as O-glycosides.

The most preferred isoflavones in the compositions of the invention are genistein, daidzein and glycitein (or as glucons, i.e. in glycosylated form, which are known as genistin, daidzin and glycitin, respectively). It is especially preferred that the isoflavones are selected from genistin (glucon), daidzin (glucon) and glycitin (glucon). Preferably the composition comprise genistein (or genistin), daidzein (or daidzin) and glycitein (or glycitin). Although the isoflavones can be applied in a wide range of ratios the best results were obtained when the genistein and daidzein were used in weight ratios of 2:1 to 1:2.

The isoflavones are preferably derived from natural sources, typically plant materials - including extracts and/or concentrates thereof. Preferred sources of the isoflavones are legumes, such as soy, chick peas and/or clover, and extracts and/or concentrates thereof. It is preferred that the plant source is a plant or plant material other than hops or extracts thereof - hops do not actually synthesise these isoflavones since the relevant metabolic pathway is not active.

### Compositions

Compositions of the present invention comprise hops isoalpha acids and isoflavones, as described above, as active agents.

The active agents can be combined with a pharmaceutically acceptable carrier or diluent to produce a systemic dosage form. Pharmaceutically acceptable diluents or carriers suitable for use in such compositions are well known in the art of pharmacy.

The systemic dosage form may consist of solid dosage forms such as tablets, hard gelatin capsules, soft gelatin capsules, bulk powders, and microcapsules of the actives. Alternately, it may consist of a liquid dosage form such as an aqueous or non-aqueous solution, emulsion, or suspension.

Solid dosage forms for oral administration are preferred compositions of the invention. Solid dosage forms are preferably prepared in unit dosage form, such as in the form of tablets and capsules. Suitably tablets may be prepared by mixing the active combination with an inert diluent such as calcium phosphate in the presence of disintegrating agents, for example maize starch, and lubricating agents, for example magnesium stearate, and tableting the mixture by known methods. Such tablets may, if desired, be provided with enteric coatings by known methods, for example by the use of cellulose acetate phthalate. Similarly, capsules, for example hard or soft gelatin capsules, containing the active combination optionally in the form of beads with or without added excipients, may be prepared by conventional means and, if desired, provided with enteric coatings in a known manner. The tablets may be formulated in a manner known to those skilled in the art so as to give a controlled release of the compound of the present invention.

Controlled release forms of the dosage forms of the present invention include rapid release formulations such as soluble granules or melt filled fast release capsules, delayed release formulations such as tablets provided with enteric coatings, for example, of cellulose acetate phthalate and, in particular, sustained release formulations. Numerous types of sustained release formulations are known to those skilled in the art. Typically, the active combination may be encapsulated within a release retarding coating, for example, a copolymer of cellulose ether and acrylate, or may be bound to small particles such as, for example, ion exchange resin beads. Alternatively, the active combination may be incorporated into a matrix containing a release retarding agent such as a hydrophilic gum e.g. xanthan gum, a cellulose derivative eg. hydroxypropyl methylcellulose, or a polysaccharide, wax or plastics material.

The active combination may be formulated into a solid dosage form in which the two active ingredients are kept separate. For example, the dosage form may be a bilayer tablet in which the active ingredients are contained in different layers. The different layers can be formulated so as to provide the optimum release profile for each drug.

Liquid fill compositions for example viscous liquid fills, liquid paste fills or thixotropic liquid fills are also suitable for oral administration. Melt filled compositions may be obtained by mixing the active combination with certain esters of natural vegetable oil fatty acids, for example, the Gelucire^{™} range available from Gattefosse to provide a variety of release rates.

Dosage forms suitable for parenteral administration can be prepared by combination of the active(s) with known pharmaceutical forms for such administration, for example sterile suspensions or sterile solutions of the active in a suitable solvent such as saline.

The preferred mode of administration is orally.

Another means of systemic dosing comprises dosing any of the aforementioned compositions in a food product which does not therefore necessarily typically include a pharmacologically acceptable carrier.

As used herein, the term "food products" includes both food products as such and beverages. Suitable food products as such include spreads, such as margarines, dairy products (including milk and yoghurts), mayonnaises desserts, convenience foods/snacks, fillings, confectioneries, health bars, breakfast cereals and cereal bars, ready-cook meals, bread and frozen confections such as ice creams, water ices and sorbets and yoghurt ice creams. Food products also include dietary/nutritional supplements. Suitable beverages include tea, tea-flavoured drinks, coffee, soft drinks (e.g. carbonated squashes etc), fruit juice and health drinks.

The food products are typically supplemented with the active ingredients of the invention so that they contain higher amounts of the active ingredient(s) than they would normally contain.

The amount of hops isoalpha acids and isoflavones present in the composition will vary depending on the type of formulation. For example, an oral dosage form such as a nutritional supplement or pharmaceutical composition will generally comprise a greater percentage of the active ingredients than a food product to achieve the desired dose. Thus, typically, an oral dosage form will comprise from 0.01 to 99 wt% of the hops isoalpha acids and isoflavones, preferably from 0.1 to 50 wt%. Preferably, the total amount of hops isoalpha acids and isoflavones per dosage form is at least 10 or 20 mg, more preferably at least 50, 100 or 200 mg.

A food product of the invention typically comprises from 0.01 wt% to 1 wt% of the hops isoalpha acids and isoflavones, the wt% being lower than in a nutritional supplement or other oral dosage form due to the greater mass ingested for a typical food product than a supplement or tablet. Preferably, the total amount of hops isoalpha acids and isoflavones per serving is at least 10 or 20 mg, more preferably at least 50, 100 or 200 mg. A serving may constitute the entire food product, as in the case of a confectionery product or individually portioned product, or a proportion of the food product.

The weight ratio of hops isoalpha acids to isoflavones can vary over a wide range. However, it is generally preferred to have a weight ratio in the range from 1:50 to 50:1, preferably ratios of 1:20 to 20:1, more preferably ratios of 1:6 to 6:1 and even more preferably from 1:4 to 4:1 and most preferably from 1:2 to 2:1 or 1:2 to 1:1, calculated as aglucon (i.e. where the glycosylated forms of isoflavones are used, the molecular weight of sugar moieties is disregarded).

By way of example, a food product of the invention may contain a blend wherein the hops alpha acid content is present in amounts of from 10 mg to 200 mg per RDI (recommended daily intake), while the isoflavones are present in amounts of from 10 mg to 200 mg per RDI. In this way the health components can be delivered as part of the daily servings of the food product.

According to another embodiment of the invention the invention also concerns food products containing a health component (= functional food) wherein the food product comprises an amount of the composition according to the present invention, so that the total recommended daily intake (= RDI) of the health components is delivered by one or more servings, such as two, three four or five servings, per day of the food product.

In these food products 20 to 400 mg, for example, recommended daily intake of the synergistic compositions according to the invention can be present. Because of the synergistic interaction between the hops isoalpha acids and the isoflavones, the food product can contain less of the individual actives, than otherwise would be required to achieve similar effects. In this way the performance of the food product is not negatively affected by the presence of the excessive amounts of the synergistic blend of active health components while the health benefits are obtained.

In addition to the above components the blends and the food products can contain other micronutrients, examples thereof being antioxidants (vitamin C or vitamin E), other vitamins in particular vitamin B1, B6 and B12, vitamin K, folic acid, minerals like calcium, magnesium, iron, copper, or zinc, however, emulsifiers also can be present as well as minor amounts of polyunsaturated fatty acids in particular DHA and EPA and in particular (deodorised) fish oils or concentrates thereof.

### Uses

A combination of hops isoalpha acids and particular isoflavones as hereinbefore defined, such as are present in compositions and food products of the present invention can be used to achieve certain health effects, in particular certain cosmetic effects. In particular, we have now found that the isoalpha acids found in hops and hop extracts can act synergistically in combination with these isoflavones in down regulating the production of prostaglandin E₂, part of the inflammatory response, following oxidative stress. Accordingly, a combination of hops isoalpha acids and isoflavones can be used to achieve anti-ageing and anti-inflammatory effects. Specifically, a combination of hops isoalpha acids and isoflavones can be used to inhibit the production of prostaglandin E₂ in response to stress, and/or to increase procollagen-1 synthesis in the skin and/or to increase decorin synthesis in the skin.

Therefore the present invention also concerns the use of a composition comprising one or more hops isoalpha acids and one or more isoflavones as hereinbefore defined, wherein the composition or food product is applied to achieve cosmetic effects, in particular skin benefits and skin related effects such as anti-ageing effects or for promoting the formation of procollagen/collagen or for promoting the decorin formation in the skin. Further the invention concerns the use of a health composition comprising hops isoalpha acids and isoflavones wherein the health composition is the blend according to the invention and wherein the blend is applied for the production of a functional food with anti-inflammatory properties with a synergistic effect on the anti-inflammatory and related health properties.

The present invention will now be further described with reference to the following examples which are illustrative only and non-limiting.

### EXAMPLES

### Example 1 - Hops isoalpha acids increase procollagen and decorin synthesis in skin cells

### Human Dermal Fibroblast Culture

Dermal fibroblasts were explanted from neonatal human foreskin from donors aged 6 months to 6 years. Cells were cultured in Dulbecco's Modified Eagle's Medium (DMEM) supplemented with 20% FCS at 37°C in a 5% CO₂ atmosphere. The culture was harvested at first passage (P1) and the bulk of the cells stored in cryogenic vials in liquid nitrogen for subsequent expansion and use at P2.

### Preparation of Fibroblast Cultures for Screening of Test Compounds

Human neonatal foreskin fibroblasts at second passage (P2) were seeded into 175 cm² flasks or 12-well plates at a seeding density of 10,000 cells/cm² in DMEM supplemented with 10% FCS. The flask was incubated at 37°C in a 5% CO₂/4% O₂ atmosphere. After 24 hours incubation the cell monolayers were washed with serum free DMEM and incubated with fresh serum free DMEM for a further 72 hours. The fibroblasts were washed again with serum free DMEM before addition of test compounds. Majority of work was carried out in cell lines 500, 501, 502 and 386.

### Test compound preparation and addition to cells

The Hops extracts were prepared as mg/ml stocks in 100% ethanol. Dimethylsulphoxide (DMSO), was rarely used as a solvent (as it can act as an anti-inflammatory agent in high enough concentrations). To avoid this effect where it was necessary to use DMSO, compounds were prepared as 100 mM stocks and it was ensured that cells were not exposed to DMSO concentrations greater than 0.1%.

Compounds were added in triplicate to the 12-well plates (0.4 ml/well). The fibroblast monolayers were incubated with the active ingredients for 24 hours at 37°C in a 5% CO₂/4% O₂ atmosphere. After 24 hours incubation, the supernatant from the triplicate treated wells was pooled and place on ice for immediate assay or snap frozen in liquid nitrogen for storage at -80°C for analysis at a later date.

### Counting Cells

Cells remaining in the wells after treatment were counted for signal normalisation purposes. Cells numbers may vary due to different treatments due to toxicity (which generally reduces cell numbers) or proliferative/mitogenic action (which increases cell number) and consequently affect the levels of procollagen-1 and decorin secretion. The remaining adherent cells were treated with 0.5 ml 1x trypsin for 15 minutes at 37°C after which time the trypsin was neutralised with 0.5 ml DMEM supplemented with 10% FCS. Triplicate wells of cells were pooled and 0.5 ml of pooled cells were added to 9.5 ml lsoton II in a counting vessel. Cell numbers were determined using a Coulter counter (Beckman), counting twice, adding the numbers together and multiplying by 20 to give the number of cells/ml.

### Dot Blot Analysis of Procollagen-1 and Decorin in fibroblast conditioned medium

The untreated and treated pooled conditioned medium from the 12-well plates was applied in triplicate to Immobilon-P transfer membrane using 96-well Bio-Dot apparatus from Biorad as described in the manufacturer's guidelines. When testing for decorin the samples were vortex mixed gently and applied directly to the membrane in triplicate. 190 µl of sample was added per well. When testing for procollagen-1 a further denaturation step was required. 60 µl of 200 mM DTT and 6 µl 10% SDS was added and the samples heated to 80°C for 2 minutes and briefly vortex mixed before adding 190 µl in triplicate to the membrane in the dot blot apparatus. The samples were allowed to filter through the membrane under gravity, allowing the protein to adhere to the membrane and the media to be removed after which the membrane was washed three times with PBS (twice under gravity and once using suction). The membrane was removed from the apparatus and blocked in blocking buffer at room temperature for one hour (5% w/v dried skimmed milk powder dissolved in PBS/0.05% Tween 20).

The membranes were probed for decorin and procollagen-1 content with diluted primary antibody (1/3333 anti-decorin in PBS/0.05% Tween 20, 1/10000 anti-procollagen-1 in 5% w/v dried skimmed milk/PBS/0.05% Tween 20) overnight at room temperature. The membranes were washed three times with PBS/0.05% Tween 20 for 20 minutes per wash before being incubated with 125 I conjugated antibodies (anti-mouse diluted 1/333 in PBS/0.05% Tween 20 for decorin and anti-rat diluted 1/1000 for procollagen-1 in 5% w/v dried skimmed milk/PBS/0.05% Tween 20) at room temperature for 2 hours. The membranes were washed three times with PBS/0.05% Tween 20 for 20 minutes per wash and allowed to air dry briefly. The membranes were wrapped in Saran® wrap, wrinkles smoothed out and exposed to a Molecular Dynamics storage phosphor screen for at least 18 hours.

### Analysis and Quantification of Procollagen-1 and Decorin Synthesis

The exposed screen was scanned by the Molecular Dynamics Phosphorimager SF using ImageQuant® software. Signal intensity was quantified by computer-assisted image analysis using the quantification tools in ImageQuant®. The intensities of the signals emanating from the test samples were related to the signals emitted from the control samples. The effects of test compounds upon decorin and procollagen-1 secretion were expressed relative to a vehicle control value of 100.

### Results

The results shown in tables 1 and 2 demonstrate that various hops isoalpha acids, reduced and unreduced stimulate expression of decorin and procollagen-1.

**Table 1 - Decorin synthesis**

| | Decorin synthesis % relative to control |
|---|---|
| Control | 100 |
| Hops Rho acids 100 µg/ml | 252 |
| Hops Isoalpha acids 100 µg/ml | 186 |
| Hops Tetrahydro-isoalpha acids 100 µg/ml | 170 |

**Table 2 - Procollagen-1 synthesis**

| | Procollagen-1 synthesis % relative to control |
|---|---|
| Control | 100 |
| Hops Rho acids 1 µg/ml | 178 |
| Hops Isoalpha acids 1 µg/ml | 134 |
| Hops Tetrahydro-isoalpha acids 10 µg/ml | 201 |

### Example 2 - Hops isoalpha acids act synergystically to inhibit prostaglandin E₂ expression in skin fibroblasts in response to stress.

### Fibroblasts PGE₂ Assay

Prostaglandin E₂ (PGE₂) production by human skin fibroblasts can be induced by the inflammatory stimulus PMA (phorbal myristate acetate). PMA represents and external stressor which induces oxidative stress and inflammatory responses in cells. This model is used to model inflammation *in vivo*.

Primary human foreskin fibroblasts at passage 2 (P2) were seeded into 96-well plates at 35,000 cells/well and maintained for 24 hours in an atmosphere of 5% carbon dioxide in Dulbecco's Modified Eagle Medium (DMEM) supplemented with 10% foetal calf serum. Novasoy 40 ^{R} containing 20 mM isoflavones, genistin, daidzin and glycetin (in a ratio of 1:1.3:0.3) and 5 mM hops isoalpha acids, either independently or in combination added to the cells (DMEM, supplemented with 10% foetal calf serum) in dimethylsulphoxide (ethanol, final concentration 1%) in triplicate and incubated for a further 24 hours. Phorbal myristate acetate (PMA) (Sigma) was added to the media and the cells incubated for a further 24 hours. The control did not contain any test compounds nor any PMA. The fibroblasts/media were then analysed immediately or snap frozen in liquid nitrogen and stored at -70°C for future analysis. The cells were then counted and data from the dot-blot analysis subsequently standardised to cell number.

Prostaglandin E2 (PGE₂) assay: Volumes of 50 µl culture medium were taken for PGE₂ assay after gently shaking the culture plate. PGE₂ levels in the medium were determined with a Biotrak PGE₂ immunoassay kit (Amersham, UK). The assay is based on the competition between unlabelled PGE2 in the sample and a fixed quantity of horseradish peroxidase labeled PGE₂ for a limited amount of fixed PGE₂ specific antibody. Concentrations of unlabeled sample PGE₂ are determined according to a standard curve, which was obtained at the same time.

When hops isoalpha acids and Novasoy 40 are added to cells at sub-optimal concentrations (i.e. those concentrations just below the concentrations that produce maximal down-regulation of PGE₂) the down-regulation of PGE₂ can be enhanced when hops isoalpha acids and 5.4 µg/ml of isoflavones from Novasoy 40 are combined at a concentration of 300 µM (isoflavones), respectively. A synergistic interaction was observed between Novasoy 40 and the hops isoalpha acids when they were combined at the above concentrations, as shown in Table 3:

**Table 3**

| | PGE2 ng/ml | PGE2 % total |
|---|---|---|
| Control | 1246.1 | - |
| Control + vehicle | 1191.6 | - |
| Control + vehicle +PMA | 12800.0 | 100.0 |
| Isoalpha acids 300 µg/ml | 3851.0 | 30.1 |
| Novasoy 5.4 µg/ml | 12800.0 | 100.0 |
| Isoalpha acids (1/1000)/ Novasoy 20 µM | 1164.5 | 9.1 |

### Example 3 - Soup Composition

3.4 grams of vegetable fat }
0.5 g of modified egg yolk } together named "creamer"
6.0 g of maltodextrin }
0.025 grams of Novasoy 40 ^{R} containing 40 wt % of the isoflavones genistin / daidzin and glycitin in a weight ratio of 1.3 : 1 : 0.3
0.000446 grams of hops isoalpha acids
0.6 grams of maize starch croutons
16.1 grams of dried potato starch
1.0.grams of salt
0.3 grams of onion solids
0.7 grams of onions
0.2 grams of parsley and herb extract
3.2 grams of flavouring agents

The creamer and the other components are mixed in mixer The blend obtained is a dried instant onion soup that can be used for making a soup by mixing it with 200 ml of boiling water under stirring.

## Claims

1. A composition comprising hops isoalpha acids and one or more isoflavones selected from genistein, genistin, daidzein, daidzin, glycitein and glycitin, wherein the weight ratio of hops isoalpha acid extract to isoflavones is from 1:50 to 50:1, calculated as aglucon.

2. A composition according to claim 1 wherein the weight ratio of hops isoalpha acids to said isoflavones is from 20:1 to 1:20, calculated as aglucon.

3. A composition according to claim 1, wherein the weight ratio of hops isoalpha acids to said isoflavones is in the range from 1:2 to 2:1, calculated as aglucon.

4. A composition according to any one of the preceding claims which comprises genistein/genistin and daidzein/daidzin present in a weight ratio of from 2:1 to 1:2, calculated as aglucon.

5. A composition according to any one of the preceding claims, wherein the isoflavones are derived from soy.

6. A composition according to any one of the preceding claims wherein the hops isoalpha acids are selected from unreduced isoalpha acids, dihydro-isoalpha acids and tetrahydro-isoalpha acids, and mixtures thereof.

7. A dosage form comprising a composition according to any one of the preceding claims, wherein the dosage form comprises from 10 mg to 200 mg of said hops isoalpha acids and from 10 mg to 200 mg of said isoflavones.

8. A food product which comprises a composition according to any one of claims 1 to 6.

9. A food product according to claim 8, wherein the amount of said hops isoalpha acids and said isoflavones present in the food product is from 20% to 100% of the total recommended daily intake (= RDI amount) of the hops isoalpha acids and isoflavones per serving.

10. A food product according to claim 8, wherein the food product is selected from the group consisting of spreads, margarines, creams, sauces, dressings, mayonnaises, ice creams, fillings, confectioneries, health bars, cereals and health drinks.

11. A food product according to any one of claims 8 to 10 wherein the food product contains 20 mg to 400 mg of a combination of said hops isoalpha acids and said isoflavones, per recommended serving.

12. A composition comprising a combination of hops isoalpha acids and one or more isoflavones selected from genistein, genistin, daidzein, daidzin, glycitein and glycitin for use in promoting the formation of collagen and/or decorin in the skin of an animal or human.

13. A composition comprising a combination of hops isoalpha acids and one or more isoflavones selected from genistein, genistin, daidzein, daidzin, glycitein and glycitin for use in reducing the effects of ageing on the skin of an animal or human.

14. A composition comprising a combination of hops isoalpha acids and one or more isoflavones selected from genistein, genistin, daidzein, daidzin, glycitein and glycitin for use in treating or preventing the effects of inflammation in an animal or human.

## Patentansprüche

1. Zusammensetzung, umfassend Hopfen-Isoalphasäuren und ein oder mehrere Isoflavone, ausgewählt aus Genistein, Genistin, Daidzein, Daidzin, Glycitein und Glycitin, wobei das Gewichtsverhältnis von Hopfen-Isoalphasäure-Extrakt zu Isoflavonen 1:50 bis 50:1, berechnet als Aglucon, ist.

2. Zusammensetzung nach Anspruch 1, wobei das Gewichtsverhältnis von Hopfen-Isoalphasäuren zu den genannten Isoflavonen 20:1 bis 1:20, berechnet als Aglucon, ist.

3. Zusammensetzung nach Anspruch 1, wobei das Gewichtsverhältnis von Hopfen-Isoalphasäuren zu den genannten Isoflavonen im Bereich von 1:2 bis 2:1, berechnet als Aglucon, liegt.

4. Zusammensetzung nach einem der vorangehenden Ansprüche, die Genistein/Genistin und Daidzein/Daidzin umfasst, die in einem Gewichtsverhältnis von 2:1 bis 1:2, berechnet als Aglucon, vorliegen.

5. Zusammensetzung nach einem der vorangehenden Ansprüche, wobei die Isoflavone aus Soja stammen.

6. Zusammensetzung nach einem der vorangehenden Ansprüche, wobei die Hopfen-Isoalphasäuren aus nicht-reduzierten Isoalphasäuren, Dihydroisoalphasäuren und Tetrahydroisoalphasäuren und Gemischen davon ausgewählt sind.

7. Dosierungsform, umfassend eine Zusammensetzung nach einem der vorangehenden Ansprüche, wobei die Dosierungsform 10 mg bis 200 mg der genannten Hopfen-Isoalphasäuren und 10 mg bis 200 mg der genannten Isoflavone umfasst.

8. Nahrungsmittelprodukt, das eine Zusammensetzung nach einem der Ansprüche 1 bis 6 umfasst.

9. Nahrungsmittelprodukt nach Anspruch 8, wobei die Menge der genannten Hopfen-Isoalphasäuren und der genannten Isoflavone, die in dem Nahrungsmittelprodukt vorliegt, 20 % bis 100 % der gesamten empfohlenen Tagesaufnahme (recommended daily intake = RDI-Menge) der Hopfen-Isoalphasäuren und Isoflavone pro Portion ist.

10. Nahrungsmittelprodukt nach Anspruch 8, wobei das Nahrungsmittelprodukt aus der Gruppe, bestehend aus Aufstrichen, Margarinen, Sahne, Soßen, Dressings, Mayonnaisen, Speiseeis, Füllungen, Konfekt, Gesundheitsriegeln, Cerealien und Gesundheitsgetränken, ausgewählt ist.

11. Nahrungsmittelprodukt nach einem der Ansprüche 8 bis 10, wobei das Nahrungsmittelprodukt 20 mg bis 400 mg einer Kombination der genannten Hopfen-Isoalphasäuren und der genannten Isoflavone pro empfohlene Portion enthält.

12. Zusammensetzung, umfassend eine Kombination von Hopfen-Isoalphasäuren und einem oder mehreren Isoflavonen, ausgewählt aus Genistein, Genistin, Daidzein, Daidzin, Glycitein und Glycitin, zur Verwendung bei der Förderung der Bildung von Kollagen und/oder Decorin in der Haut eines Tiers oder Menschens.

13. Zusammensetzung, umfassend eine Kombination von Hopfen-Isoalphasäuren und einem oder mehreren Isoflavonen, ausgewählt aus Genistein, Genistin, Daidzein, Daidzin, Glycitein und Glycitin, zur Verwendung bei der Reduzierung der Effekte der Alterung an der Haut eines Tiers oder eines Menschens.

14. Zusammensetzung, umfassend eine Kombination aus Hopfen-Isoalphasäuren und einem oder mehreren Isoflavonen, ausgewählt aus Genistein, Genistin, Daidzein, Daidzin, Glycitein und Glycitin, zur Verwendung bei der Behandlung oder Prävention der Wirkungen einer Entzündung bei einem Tier oder einem Menschen.

## Revendications

1. Composition comprenant des acides isoalpha de houblons et une ou plusieurs isoflavones choisies parmi la génistéine, la génistine, la daidzéine, la daidzine, la glycitéine et la glycitine, dans laquelle le rapport de poids de l'extrait d'acide isoalpha de houblon aux isoflavones est de 1/50 à 50/1, calculé en aglucone.

2. Composition selon la revendication 1, dans laquelle le rapport de poids des acides isoalpha de houblons auxdites isoflavones est de 20/1 à 1/20, calculé en aglucone.

3. Composition selon la revendication 1, dans laquelle le rapport de poids des acides isoalpha de houblons auxdites isoflavones est dans la fourchette de 1/2 à 2/1, calculé en aglucone.

4. Composition selon l'une quelconque des revendications précédentes, qui comprend la génistéine/génistine et la daidzéine/daidzine présentes dans un rapport de poids de 2/1 à 1/2, calculé en aglucone.

5. Composition selon l'une quelconque des revendications précédentes, dans laquelle les isoflavones sont dérivées du soja.

6. Composition selon l'une quelconque des revendications précédentes, dans laquelle les acides iso alpha de houblons sont choisis parmi les acides iso alpha non réduits, les acides dihydro iso alpha et les acides tétrahydro iso alpha, et leurs mélanges.

7. Composition selon l'une quelconque des revendications précédentes, dans laquelle la forme pharmaceutique comprend de 10 mg à 200 mg desdits acides iso alpha de houblons et de 10 mg à 200 mg desdites isoflavones.

8. Produit alimentaire qui comprend une composition selon l'une quelconque des revendications 1 à 6.

9. Composition alimentaire selon la revendication 8, dans laquelle la quantité desdits acides isoalpha de houblons et desdites isoflavones présents dans le produit alimentaire est de 20% à 100% de la prise journalière totale recommandée (= quantité RDI) des acides iso alpha de houblons et des isoflavones par portion.

10. Composition alimentaire selon la revendication 8, dans laquelle le produit alimentaire est choisi parmi le groupe composé de produits à tartiner, de margarines, de crèmes, de sauces, d'assaisonnements, de mayonnaises, de crèmes glacées, de garnitures, de confiseries, de barres diététiques, céréales et boissons diététiques.

11. Composition alimentaire selon l'une quelconque des revendications 8 à 10, dans laquelle le produit alimentaire contient de 20 mg à 400 mg d'une combinaison desdits acides iso alpha de houblons et desdites isoflavones, par portion recommandée.

12. Composition comprenant une combinaison d'acides iso alpha de houblons et une ou plusieurs isoflavones choisies parmi la génistéine, la génistine, la daidzéine, la daidzine, la glycitéine et la glycitine pour une utilisation dans la promotion de la formation de collagène et/ou de décorine dans la peau d'un animal ou d'un humain.

13. Composition comprenant une combinaison d'acides iso alpha de houblons et une ou plusieurs isoflavones choisies parmi la génistéine, la génistine, la daidzéine, la daidzine, la glycitéine et la glycitine pour une utilisation dans la réduction des effets du vieillissement sur la peau d'un animal ou d'un humain.

14. Composition comprenant une combinaison d'acides iso alpha de houblons et une ou plusieurs isoflavones choisies parmi la génistéine, la génistine, la daidzéine, la daidzine, la glycitéine et la glycitine pour une utilisation dans le traitement ou la prévention des effets de l'inflammation chez un animal ou un humain.
